# EUROPEAN PATENT SPECIFICATION

(11) **EP 4 539 897 B1**
(45) Date of publication and mention of the grant of the patent: **21.01.2026**
(21) Application number: 23734482.5
(22) Date of filing: 15.06.2023
(51) Int. Cl.: A61L 9/20

(54) **LED FILAMENT COMPRISING LEDS ARRANGED TO EMIT VIOLET AND UV LIGHT**
LED-FADEN MIT LEDS, DIE VIOLETTES UND ULTRAVIOLETTES LICHT EMITTIEREN
FILAMENT À DEL COMPRENANT DES DEL AGENCÉES POUR ÉMETTRE DE LA LUMIÈRE VIOLETTE ET UV

(30) Priority: 20.06.2022 EP 22179821
(43) Date of publication of application: 23.04.2025
(73) Proprietor: Signify Holding B.V., 5656 AE Eindhoven (NL)
(72) Inventor: VAN BOMMEL, Ties, 5656 AE Eindhoven (NL)
(74) Representative: Verweij, Petronella Daniëlle
(86) International application number: PCT/EP2023/066117
(87) International publication number: WO 2023/247320

(56) References cited:
- WO-A1-2021/094257
- WO-A1-2022/005505
- CN-A- 111 029 452
- CN-U- 203 842 065
- KR-B1- 102 404 462
- US-A1- 2019 234 563

## Description

### FIELD OF THE INVENTION

The present invention generally relates to light emitting diode, LED, filaments. More specifically, the present invention is related to LED filaments arranged to emit violet light and ultraviolet, UV, light.

### BACKGROUND OF THE INVENTION

The use of light emitting diodes (LED) for illumination purposes continues to attract attention. Compared to incandescent lamps, fluorescent lamps, neon tube lamps, etc., LEDs provide numerous advantages such as a longer operational life, a reduced power consumption, and an increased efficiency related to the ratio between light energy and heat energy. In particular, LED filament lamps are highly appreciated as they are very decorative.

Due to the advantageous aspects of the use of LEDs, the interest has rapidly increased to replace conventional light sources with LEDs in many lighting arrangements. It will be appreciated that this replacement, also called retrofitting, is appreciated and desired by users who wish to have the look of an incandescent bulb. The light source replacement (retrofitting) is often performed by removing the conventional light source(s) from the luminaire (e.g. a lamp holder) of the lighting arrangement and attaching the LEDs, LED arrangement(s) or LED device(s) into the luminaire. One of these concepts is based on LED filaments which are placed in a bulb, as the appearance of lamps of this kind are appreciated as they are highly decorative.

Furthermore, it is of interest to combine the advantageous properties of LED filaments with respect to aesthetics and light distribution purposes according to the above with the advantageous properties of disinfection (bactericidal) lighting. It will be appreciated that disinfection lighting has become a topic of renewed interest as the demand for sterilization increases. For example, UV (100-380 nm) and/or violet light (380-420 nm) can be used for disinfection purposes, e.g. inactivating/killing bacteria.

It should also be noted that it is a wish to replace older technologies (e.g. mercury-based UV tubes) with UV LED-based solutions. Furthermore, existing arrangements comprising LEDs arranged to emit UV light may suffer from complexity and/or operational issues regarding efficiency characteristics and/or safety.

Hence, it is an object of the present invention to combine the advantageous properties of LEDs with respect to energy efficiency and light distribution purposes with the advantageous properties of disinfection (bactericidal and/or viricidal) lighting. It is also an object of the present invention to overcome at least some complexity and/or operational issues regarding efficiency characteristics and/or safety. Document CN 203 842065 U discloses an LED disinfection device using UV light; document KR 102 404 462 B1 discloses a module for sterilization, including UV-C LED light sources and violet LED light sources.

### SUMMARY OF THE INVENTION

It is of interest to combine the advantageous properties of LEDs with respect energy efficiency, light distribution purposes and/or aesthetics with the advantageous properties of providing disinfection (bactericidal and/or viricidal) lighting, whilst providing an efficient and/or safely operated LED arrangement.

This and other objects are achieved by providing a LED filament having the features in the independent claim. Preferred embodiments are defined in the dependent claims.

Hence, according to the present invention, there is provided a light emitting diode, LED, filament, configured to emit LED filament light. The LED filament comprises an elongated carrier extending in a first direction, A, comprising a first surface and a second surface oppositely arranged the first surface. The LED filament further comprises a first linear array of a plurality of first light emitting diodes, LEDs, arranged on the first surface, wherein the plurality of first LEDs is arranged to emit ultra-violet, UV, light with a first centroid wavelength, λ_{C1}, in a wavelength range of 100-380 nm. The LED filament further comprises a second linear array of a plurality of second LEDs, arranged on the second surface, wherein the plurality of second LEDs is arranged to emit violet light with a second centroid wavelength, λ_{C2}, in a wavelength range of 380-420 nm.

Thus, the present invention is based on the idea of providing a LED filament comprising a first linear array of LEDs arranged on a first surface configured to emit UV light and a second linear array of LEDs arranged on a second surface configured to emit violet light. The first and second linear array are arranged on two different surfaces of the same carrier, wherein the second surface is oppositely arranged the first surface. In other words, the first linear array emits UV light in a direction opposite the direction in which the second linear array of LEDs emit violet light. Hence, there is provided a LED filament which is able to efficiently, and safely, provide visible, decorative and aesthetically pleasing violet light while providing disinfection lighting, i.e. UV light.

The present invention is advantageous in that the linear arrays of the plurality of LEDs may provide a line emission of violet light and a line emission of UV light respectively, in primarily opposite directions.

The present invention is further advantageous in that the visible light may be primarily emitted in one direction, while the possibly harmful UV light may be emitted in a secondary direction.

The present invention is further advantageous in that more than one side of the carrier of the LED filament is used. Consequently, the LED filament may be smaller and/or more convenient.

The present invention is further advantageous in that the visible violet light may be decorative and aesthetically pleasing, and simultaneously indicate that UV light is being emitted. As UV light may be harmful to people, the violet light emitted by the plurality of first LEDs provides an increased safety.

The present invention is advantageous in that the linear arrays of the plurality of LEDs of the LED filament allow for a non-complex and convenient electric circuitry. In turn, this increases the service life of the LED filament and/or reduces the risk of malfunction thereof at operation.

The present invention is advantageous in that the LEDs are relatively small, and the LED filaments providing UV light may be smaller than conventional UV lights. As a consequence, the present UV LED filaments may be more convenient and versatile.

It will be appreciated that that the wavelength, 380-420 nm, of the second LEDs provides both visible light and at least partially disinfecting light.

It will be further appreciated that the first LEDs provide UV light with disinfecting properties.

It will be further appreciated that the LED filament of the present invention furthermore comprises relatively few components. The relatively low number of components is advantageous in that the LED filament is relatively inexpensive to fabricate. Moreover, the relatively low number of components of the LED filament implies an easier recycling, especially compared to devices or arrangements comprising a relatively high number of components which impede an easy disassembling and/or recycling operation.

The LED filament which is configured to emit LED filament light, comprises an elongated carrier. By the term "carrier", it is here meant an element, substrate, printed circuit board, PCB, or the like, arranged to mechanically and/or electrically support LEDs. Hence, the plurality of LEDs may be arranged, mounted and/or mechanically coupled on/to the carrier (e.g. a substrate), wherein the carrier is configured to mechanically and/or electrically support the LEDs. The elongated carrier extends in a first direction, A, and comprises a first surface and second surface, wherein the second surface is oppositely arranged the first surface. The first and second surface may be the front and back side of an elongated and flat carrier. The LED filament comprises a first linear array of a plurality of first LEDs arranged on the first surface of the elongated carrier. The first LEDs are arranged to emit ultra-violet, UV, light with a first centroid wavelength, λ_{C1}, in a wavelength range of 100-380 nm. By "centroid wavelength", it is here meant a (dominant) peak wavelength, i.e. a wavelength at which the light reaches a maximum intensity. The plurality of first LEDs may be arranged to emit UV light in a wavelength range of 100-380 nm with the first centroid wavelength, λ_{C1}, anywhere in the wavelength range 100-380 nm. The LED filament further comprises a second linear array of a plurality of second LEDs arranged on the second surface of the elongated carrier. The second LEDs are arranged to emit violet light with a second centroid wavelength, λ_{C2}, in a wavelength range of 380-420 nm. The plurality of second LEDs may be arranged to emit violet light in a wavelength range of 380-420 nm with the second centroid wavelength, λ_{C2}, anywhere in the wavelength range 380-420 nm. The first surface may be free from (second) LEDs emitting violet light with a second centroid wavelength, λ_{C2}, in a wavelength range of 380-420 nm. and/or the second surface may be free from (first) LEDs emitting ultra-violet, UV, light with a first centroid wavelength, λ_{C1}, in a wavelength range of 100-380 nm. The LED filament may comprise one or more first linear arrays of first LEDs arranged on the first surface. The LED filament may comprise one or more second linear arrays of second LEDs arranged on the second surface.

In one or more embodiments, the plurality of first LEDs of the first linear array may be electrically connected in series (i.e. in a serial circuitry) and/or the plurality of second LEDs of the first linear array may be electrically connected in series (i.e. in a serial circuitry). In particular, the first linear array and the second linear array may be electrically connected in parallel.

According to an embodiment of the present invention, the first and second linear arrays extend in a direction parallel with the first direction, A.

According to an embodiment of the present invention, the LED filament further comprises a first encapsulant. The first encapsulant at least partially encloses the second linear array of the plurality of second LEDs. The first encapsulant comprises a light scattering material configured to scatter at least part of the violet light emitted from the plurality of second LEDs. It is to be understood that the first encapsulant may also enclose at least part of the second surface. By the term "encapsulant", it is here meant a material, element, arrangement, or the like, which is configured or arranged to at least partially surround, encapsulate and/or enclose the linear array. The light-scattering material is configured to scatter the light. More specifically, the light scattering material may be configured to scatter the violet light in a wavelength range of 380-420 nm. The light-scattering material may enable forward and/or backward scattering. The scattering may be primarily forward scattering or backward scattering. The light scattering material may comprise a silicone matrix with at least one of Al₂O₃, BaSO₄, TiO₂, SiO₂, CaF₂, CaCO₃, and BaTiO₃ particles. The present embodiment is advantageous in that the violet light may be more decorative and/or aesthetically pleasing.

According to an embodiment of the present invention, the elongated carrier is light transmissive, and the light scattering material is arranged to scatter at least a part of the violet light through the elongated carrier. In other words, a part of the violet light is scattered from an encapsulant, such that it changes direction and travels through the light-transmissive carrier. More specifically, the elongated carrier is light-transmissive for the violet light of the second LEDs. The elongated carrier may be light-transmissive for violet light in the wavelength range 380-420 nm while not being light transmissive for UV light in the wavelength range 100-380 nm. By the term "light-transmissive", it is here meant that the carrier comprises a material, composition and/or substance which is transparent and/or translucent, allowing light to be transmitted through the carrier. The present embodiment is advantageous in that the LED filament light may be decorative and/or aesthetically pleasing, and may simultaneously indicate, in an improved manner, where/when UV light is being emitted. A reason for this is because a part of the violet light is emitted together with the UV light, i.e. at least partially in the same direction.

According to an embodiment of the present invention, the LED filament further comprises a second encapsulant. The second encapsulant at least partially encloses the second linear array of the plurality of second LEDs. The second encapsulant comprises a reflective layer arranged on the second encapsulant to reflect at least part of the violet light emitted from the plurality of second LEDs. It is to be understood that the second encapsulant may also enclose at least part of the second surface. The present embodiment is advantageous in that the violet light may be more decorative and/or aesthetically pleasing. According to an example, the second encapsulant may also comprise light scattering material configured to scatter at least part of the violet light. The second encapsulant may be the same as the first encapsulant.

According to an embodiment of the present invention, the elongated carrier is light transmissive, and the reflective layer is arranged to reflect at least a part of the violet light through the elongated carrier. The present embodiment is advantageous in that the LED filament light may be decorative and/or aesthetically pleasing, and may simultaneously indicate, in an improved manner, where/when UV light is being emitted. A reason for this is because a part of the violet light is emitted together with the UV light, i.e. at least partially in the same direction.

According to an embodiment of the present invention, one of the first encapsulant is configured to scatter at least 55 % of the violet light through the elongated carrier, and the second encapsulant is configured to reflect at least 55 % of the violet light through the elongated carrier, is fulfilled. The present embodiment is advantageous in that the violet light is provided at least partially in the same direction as the UV light from the first LEDs. In other words, the violet light may better indicate where/when the UV light is emitted.

According to an embodiment of the present invention, the LED filament further comprises a third encapsulant at least partially enclosing the first linear array of the plurality of first LEDs, wherein the third encapsulant is light transmissive. It is to be understood that the third encapsulant may also enclose at least part of the first surface. The third encapsulant may be light-transmissive for violet light in the wavelength range 380-420 nm and/or for UV light in the wavelength range 100-380 nm. The third encapsulant may be free from a light scattering material. The third encapsulant may have some light scattering properties. For example, at most 10% of the UV light emitted from the plurality of first LEDs is scattered in the third encapsulant, preferably at most 7%, more preferably at most 5%, most preferably at most 3%. The present embodiment is advantageous in that it may improve the disinfection performance of the LED filament, since the UV light may be extracted better from the first LEDs, e.g. via better distribution of the light after it passes through the third encapsulant.

According to the present invention, the first linear array comprises a number, N₁, of first LEDs, and the second linear array comprises a second number, N₂, of second LEDs, N₁ < N₂ is fulfilled. Preferably, N₁ <= 0.5·N₂ is fulfilled. The present embodiment is advantageous in that a more efficient and aesthetically pleasing LED light distribution may be provided. A reason for this is because an improved line emission is achieved for the visible violet light, by the second linear array, by having a relatively high number of second LEDs, while the number of first LEDs emitting non-visible UV light is relatively few in order to provide a more efficient disinfecting lighting, e.g. by generating less heat while still providing sufficient lighting for disinfection.

According to an embodiment of the present invention, the number of first LEDs, N₁, per unit length, L₁, of the first linear array and the number of second LEDs, N₂, per unit length, L₂, of the second linear array fulfil N₁/L₁ < N₂/L₂. Preferably, N₁/L₁ < 0.5·N₂/L₂ is fulfilled. The present embodiment is advantageous in that a more efficient and aesthetically pleasing LED light distribution may be provided. A reason for this is because an improved line emission is achieved for the visible violet light, by the second linear array, by having a relatively high number of second LEDs per unit length, while the number of first LEDs per unit length that emits non-visible UV light is relatively low in order to provide a more efficient disinfecting lighting, e.g. by generating less heat while still providing sufficient lighting for disinfection.

According to an embodiment of the present invention, the second centroid wavelength, λ_{C2}, is in a wavelength range of 400-410 nm. The present embodiment is advantageous in that the violet light comprises light of a wavelength that is a balanced option between safety and disinfection performance.

According to an embodiment of the present invention, the first centroid wavelength, λ_{C1}, is in a wavelength range of 100-280 nm. The present embodiment is advantageous in that the UVC light in the range of 100-280 nm may penetrate the skin less than UV light of a longer wavelength, such as UVB light. Consequently, the UVC light may be less harmful than other types of UV light, for example to the skin of a person. The present embodiment is further advantageous in that the wavelength range 100-280 nm has an improved disinfection performance, i.e. an improved operation of inactivating/killing bacteria.

According to an embodiment of the present invention, there is provided a LED filament arrangement. The LED filament arrangement comprises at least one LED filament according to any one of the preceding embodiments. The LED filament arrangement further comprises a controller coupled to the set of linear arrays, wherein the controller is configured to individually control the operation of the first linear array and the second linear array. The LED filament further comprises at least one of a user interface coupled to the controller, wherein the controller is configured to be controlled by an operator via the user interface and a sensor coupled to the controller, wherein the sensor is configured to register sensor data and wherein the controller is configured to individually control the operation of the respective linear array of the set of linear arrays based on the sensor data. The present embodiment is advantageous in that the individual control of the linear array(s) as provided by the controller may even further improve the aesthetics and/or light distribution purposes with the advantageous properties of disinfection (bactericidal and/or viricidal) lighting

According to an embodiment of the present invention, the sensor is configured to detect at least one of the presence of at least one person and the distance of a person to the sensor. The sensor may comprise a motion sensor, and/or proximity/presence sensor. The present embodiment is advantageous in that the UV light may be automatically adjusted depending on whether or not a person is detected. As a consequence of this, the LED device may be operated more safely, since the UV light may be decreased and/or turned off in case a person is at risk of being illuminated with UV light.

According to an embodiment of the present invention, there is provided a tubular lighting device. The tubular lighting device comprises at least one of a LED filament according to any one of the preceding embodiments, and a LED filament arrangement according to any one of the preceding embodiments. The tubular lighting device further comprises a tubular housing. The tubular housing comprises a first end cap at a first end of the tubular housing, and a second end cap at a second end of the tubular housing, opposite the first end of the tubular housing. The first and second end caps comprise a first pair and second pair of pins, respectively. The tubular lighting device further comprises a fixture. The fixture comprises first and second connectors, wherein the first pair and second pair of pins of the tubular housing are configured for mating connection to the first and second connectors, respectively, for mechanical and electrical connection between the tubular housing and the fixture. The tubular lighting device may comprise one or more LED filaments. The present embodiment is advantageous in that the LED filament (or LED filament arrangement) according to the invention may be conveniently arranged in a tubular lighting device.

In a further embodiment of the present invention, there is provided a luminaire. The luminaire comprises one of at least one LED filament according to any one of preceding embodiments, and a LED filament arrangement according any one of the preceding embodiments. The luminaire further comprises a light transmissive cover at least partially enclosing the at least one LED filament, and an electrical connection connected to the at least one LED filament for a supply of power to the plurality of LEDs of the at least one LED filament. By the term "cover", it is here meant an enclosing element, such as a cap, cover, envelope, or the like, comprising an at least partial translucent and/or transparent material. The present embodiment is advantageous in that the LED filament (or LED filament arrangement) according to the invention may be conveniently arranged in substantially any luminaire, lamp or lighting device, such as a tubular lighting device, a LED filament lamp or a LED filament luminaire, luminaire, lighting system, or the like. The luminaire may further comprise a driver for supplying power to the LEDs of the LED filament.

Further objectives of, features of, and advantages with, the present invention will become apparent when studying the following detailed disclosure, the drawings and the appended claims. Those skilled in the art will realize that different features of the present invention can be combined to create embodiments other than those described in the following.

### BRIEF DESCRIPTION OF THE DRAWINGS

This and other aspects of the present invention will now be described in more detail, with reference to the appended drawings showing embodiment(s) of the invention.
Fig. 1a schematically show a cross-section from a side-view of a LED filament according to an exemplifying embodiment of the present invention,
Fig. 1b schematically disclose distributions of LED light of the LED filament according to an exemplifying embodiment of the present invention,
Figs. 2a-b schematically show a top view and bottom view of a LED filament according to exemplifying embodiments of the present invention,
Figs. 3 and 4 schematically show cross sections, in the longitudinal direction, of a LED filament according to exemplifying embodiments of the present invention,
Fig. 5 schematically shows a LED filament arrangement according to an exemplifying embodiment of the present invention, and
Fig. 6 shows a tubular LED device according to exemplifying embodiments of the present invention.

### DETAILED DESCRIPTION

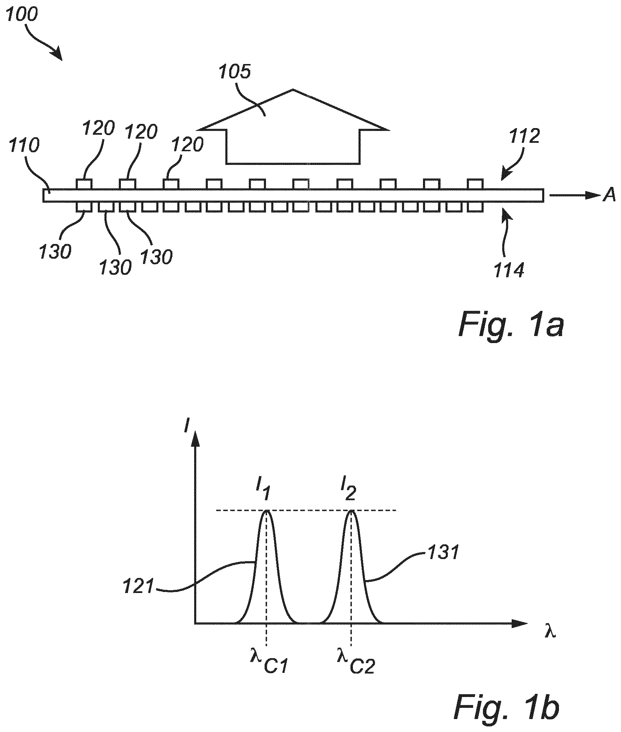

Fig. 1a schematically show a cross-section from a side-view of a LED filament 100 according to exemplifying embodiments of the present invention. The LED filament 100 is configured to emit LED filament light 105. The LED filament 100 comprises an elongated carrier 110 extending in a first direction, A. The elongated carrier 110 comprises a first surface 112 and a second surface 114, wherein the second surface is oppositely arranged the first surface 112. The first 112 and second surface 114 may be the front side and back side of an elongated and relatively flat carrier. The LED filament 100 further comprises a first linear array 120 of a plurality of first LEDs arranged on the first surface 112. The plurality of first LEDs is arranged to emit UV light with a first centroid wavelength, λ_{C1}, in a wavelength range of 100-380 nm. The LED filament 100 further comprises a second linear array 130 of a plurality of second LEDs arranged on the second surface 114. The plurality of second LEDs is arranged to emit violet light with a second centroid wavelength, λ_{C2}, in a wavelength range of 380-420 nm.

Fig. 1b schematically disclose distributions of the LED light, provided by the LED filament, with intensity (y-axis, arb. units) as a function of wavelength (x-axis, arb. units). The UV light 121 has a first centroid wavelength, λ_{C1}, in a wavelength range of 100-380 nm, and the violet light 131 has a second centroid wavelength, λ_{C2}, in a wavelength range of 380-420 nm. The first centroid wavelength, λ_{C1}, may be in a wavelength range of 100-280 nm. The second centroid wavelength, λ_{C2}, may be in a wavelength range of 400-410 nm. It is to be understood that the intensities I₁ and I₂ may be the same or different.

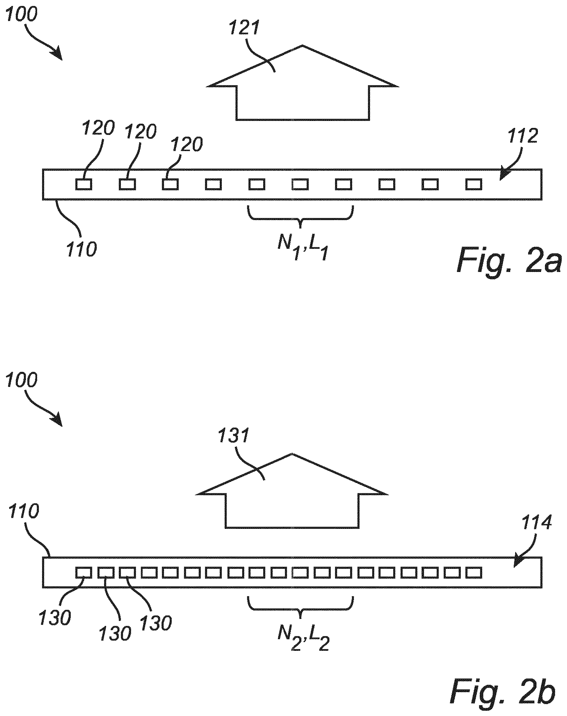

Fig. 2a-b schematically show a top view and a bottom view of a LED filament 100 according to exemplifying embodiments of the present invention. The LED filament 100 comprises an elongated carrier 110. The LED filament 100 further comprises a first linear array 120 arranged on a first surface 112 of the elongated carrier 110, and a second linear array 130 arranged on a second surface 114 of the elongated carrier 110. The first linear array 120 comprises a plurality of first LEDs arranged to emit UV light 121 with a first centroid wavelength, λ_{C1}, in wavelength range of 100-380 nm. The second linear array 130 comprises a plurality of second LEDs arranged to emit violet light 131 with a second centroid wavelength, λ_{C2}, in a range of 380-420 nm.

In Fig. 2a the first linear array 120 comprises N₁ first LEDs distributed along the elongated carrier 110 per unit length L₁, arranged on the first surface 112. In Fig. 2b the second linear array 130 comprises N₂ first LEDs distributed along the elongated carrier 110 per unit length L₂, arranged on the first surface 114. The second linear array 130 may have a higher number of LEDs, N₁ < N₂, and/or a higher number of LEDs per unit length, N₁/L₁ < N₂/L₂, compared to the first linear array 120. According to the invention, the number of first LEDs, N₁, and the number of second LEDs, N₂, fulfils N₁ <= 0.5·N₂. In addition and/or alternatively, the number of first LEDs, N₁, per unit length, L₁, of the first linear array and the number of second LEDs, N₂, per unit length, L₂, of the second linear array may fulfil N₁/L₁ < 0.5·N₂/L₂. The plurality of first LEDs may comprise at least 5 LEDs, preferably at least 10 LEDs, more preferably at least 15 LEDs, most preferably at least 20 LEDs. The plurality of second LEDs may comprise at least 10 LEDs, preferably at least 20 LEDs, more preferably at least 30 LEDs, most preferably at least 40 LEDs.

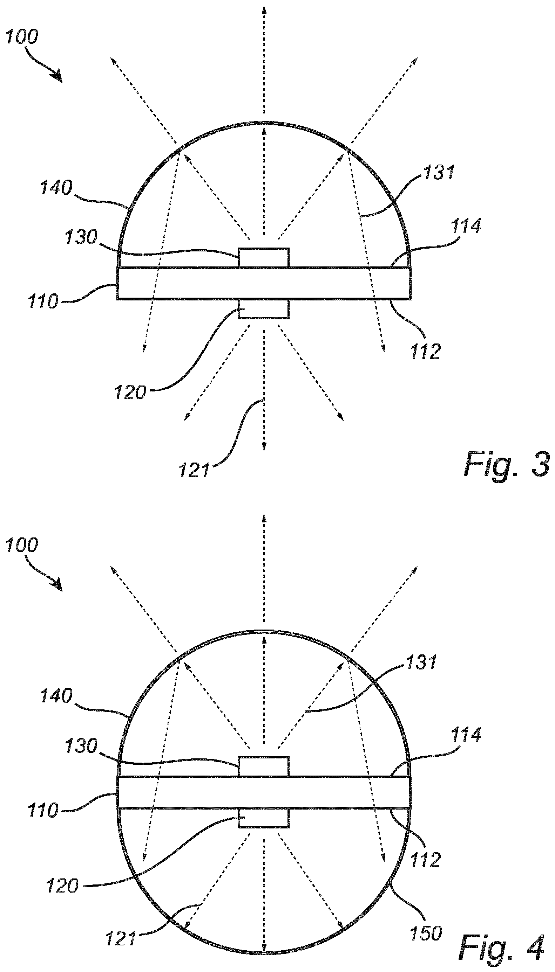

Fig. 3 schematically shows a cross section in the longitudinal direction of a LED filament 100 according to exemplifying embodiments of the present invention. It should be noted that the LED filament 100 shown in Fig. 3 has several features in common with the LED filament 100 shown in Fig. 1a and 2a-b, and it is hereby referred to Fig. 1a, 2a-b and the associated texts for an increased understanding of some of the features and/or functions of the LED filament 100. The LED filament 100 comprises an elongated carrier 110, wherein a first linear array 120 is arranged on the first surface 112 of the elongated carrier 110 and a second linear array 130 is arranged on the second surface 114 of the elongated carrier 110. The elongated carrier 110 is light transmissive. The LED filament 100 further comprises a first encapsulant 140. The first encapsulant 140 at least partially encloses the second linear array 130. The first encapsulant 140 may fully enclose the second linear array 130. Consequently, the first encapsulant 140 may enclose the second surface 114, partially or fully. The first encapsulant 140 comprises a light scattering material and/or a reflective layer. The light scattering material 140 is configured to scatter at least part of the violet light 131 from the second LEDs, wherein the scattered violet light 131 may be scattered through the light transmissive elongated carrier 110. The reflective layer is arranged to reflect at least part of the violent light 131 through the elongated carrier 110. The first encapsulant 140 may be configured to, if the first encapsulant 140 comprises a light scattering material, scatter at least 55 % of the violet light through the elongated carrier 110. The first encapsulant 140 may be configured to, if the first encapsulant 140 comprises a reflective layer, reflect at least 55 % of the violet light through the elongated carrier 110.

Fig. 4 schematically shows a cross section in the longitudinal direction of a LED filament according to exemplifying embodiments of the present invention. It should be noted that the LED filament 100 shown in Fig. 4 has several features in common with the LED filament 100 shown in Fig. 1a, 2a-b and 3, and it is hereby referred to Fig. 1a, 2a-b, 3 and the associated texts for an increased understanding of some of the features and/or functions of the LED filament 100. The LED filament 100 comprises an elongated carrier 110, a first 120 and second linear array 130 arranged on a first 112 and second surface 114, respectively, of the elongated carrier 110. The LED filament 100 comprises a first encapsulant 140 at least partially enclosing the second linear array 130. In Fig. 4, the LED filament further comprises a third encapsulant 150. The third encapsulant 150 encloses, partially or fully, the first linear array 120 of the plurality of first LEDs. The third encapsulant 150 is light transmissive. The third encapsulant 150 may be free from light scattering material.

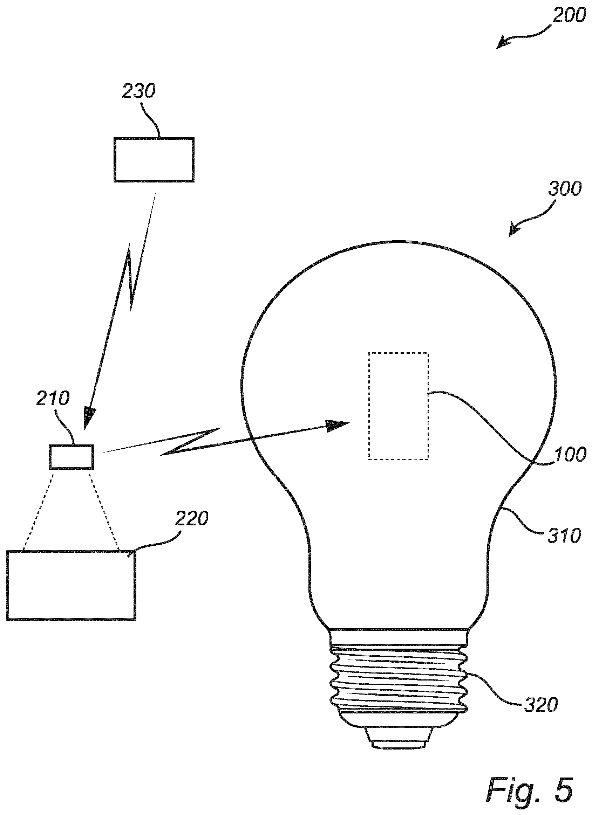

Fig. 5 schematically shows a LED filament arrangement 200 according to exemplifying embodiments of the present invention. The LED filament arrangement 200 comprises at least one LED filament 100 according to an embodiment of the present invention. The LED filament arrangement 200 may comprise a plurality of LED filaments 100. It should be noted that the LED filament 100 shown in Fig. 5 has several features in common with the LED filament 100 shown in Fig. 1a, 2a-b, 3 and 4, and it is hereby referred to Fig. 1a, 2a-b, 3, 4 and the associated texts for an increased understanding of some of the features and/or functions of the LED filament 100.

The LED filament arrangement 200 further comprises a controller 210 coupled to the set of linear arrays of the at least one LED filament 100. The set of linear arrays may be the group comprising the first and second linear array(s). The controller 210 is configured to individually control the operation of the first linear array and the second linear array. The LED filament arrangement 200 further comprises a user interface 300 coupled to the controller 210, wherein the controller 210 is configured to be controlled by an operator via the user interface 300, and/or a sensor 310 coupled to the controller, wherein the sensor is configured to register sensor data and wherein the controller 210 is configured to individually control the operation of the respective linear array of the set of linear arrays based on the sensor data. The sensor 310 may be configured to detect at least one of the presence of at least one person and the distance of a person to the sensor.

In an embodiment there is provided a luminaire 300. The luminaire 300 comprises at least one LED filament 100 according to an embodiment of the present invention. The luminaire 300 comprises a light transmissive cover 310 at least partially enclosing the at least one LED filament 100, and an electrical connection 320 connected to the at least one LED filament 100 for a supply of power to the plurality of LEDs of the at least one LED filament 100. The LED filament arrangement 200 may comprise the luminaire 300.

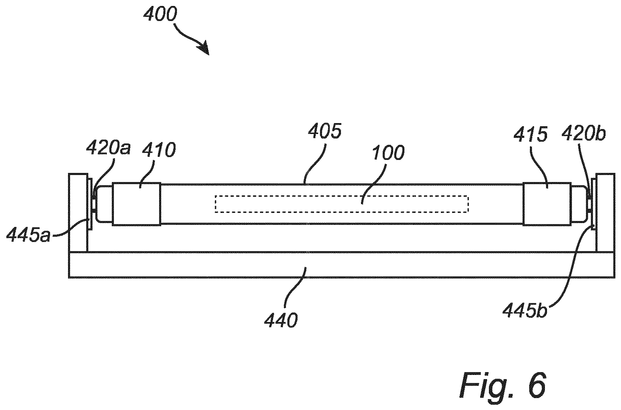

Fig. 6 shows a tubular LED device 400 according to exemplifying embodiments of the present invention. The tubular LED device, TLED, 400 comprises a LED filament 100 according to an embodiment of the present invention and/or a LED filament arrangement according to an embodiment of the present invention. The TLED 400 further comprises a tubular housing 405. The tubular housing 405 comprises a first end cap 410 arranged at a first end of the tubular housing 405, and a second end cap 415 at a second end of the tubular housing 405. The first and second end caps 410, 415 comprise a first pair and second pair of pins 420a, 420b, respectively. The TLED device 400 further comprises a fixture 440. The fixture 440 comprises first and second connectors 445a, 445b, wherein the first pair and second pair of pins 420a, 420b of the tubular housing 405 are configured for mating connection to the first and second connectors 420a, 420b, respectively, for mechanical and electrical connection between the tubular housing 405 and the fixture 440.

The person skilled in the art realizes that the present invention by no means is limited to the preferred embodiments described above. On the contrary, many modifications and variations are possible within the scope of the appended claims. For example, one or more of the LED filament 100, the elongated carrier 110, the LEDs, etc., may have different shapes, dimensions and/or sizes than those depicted/described.

## Claims

1. A light emitting diode, LED, filament (100), configured to emit LED filament light (105), comprising:
an elongated carrier (110) extending in a first direction, A, comprising a first surface (112) and a second surface (114) oppositely arranged the first surface,
a first linear array (120) of a plurality of first light emitting diodes, LEDs, arranged on the first surface, wherein the plurality of first LEDs is arranged to emit ultra-violet, UV, light (121) with a first centroid wavelength, λ_{C1}, in a wavelength range of 100-380 nm,
a second linear array (130) of a plurality of second LEDs, arranged on the second surface, **characterized in that** the plurality of second LEDs is arranged to emit violet light (131) with a second centroid wavelength, λ_{C2}, in a wavelength range of 380-420 nm, and
**in that** the first linear array comprises a number, N₁, of first LEDs, and the second linear array comprises a second number, N₂, of second LEDs, wherein N₁ <= 0.5·N₂.

2. The LED filament according to claim 1, wherein the first and second linear arrays extend in a direction parallel with the first direction, A.

3. The LED filament according to claim 1 or 2, further comprising a first encapsulant (140) at least partially enclosing the second linear array of the plurality of second LEDs, wherein the first encapsulant comprises a light scattering material configured to scatter at least part of the violet light emitted from the plurality of second LEDs.

4. The LED filament according to claim 3, wherein the elongated carrier is light transmissive, and wherein the light scattering material is arranged to scatter at least a part of the violet light through the elongated carrier.

5. The LED filament according to any one of the preceding claims, further comprising a second encapsulant (145) at least partially enclosing the second linear array of the plurality of second LEDs, wherein the second encapsulant comprises a reflective layer arranged on the second encapsulant to reflect at least part of the violet light emitted from the plurality of second LEDs.

6. The LED filament according to claim 5, wherein the elongated carrier is light transmissive, and wherein the reflective layer is arranged to reflect at least a part of the violet light through the elongated carrier.

7. The LED filament according to claims 4 or 6, wherein one of
the first encapsulant is configured to scatter at least 55 % of the violet light through the elongated carrier, and
the second encapsulant is configured to reflect at least 55 % of the violet light through the elongated carrier,
is fulfilled.

8. The LED filament according to any one of the preceding claims, further comprising a third encapsulant (150) at least partially enclosing the first linear array of the plurality of first LEDs, wherein the third encapsulant is light transmissive.

9. The LED filament according to any one of the preceding claims, wherein the number of first LEDs, N₁, per unit length, L₁, of the first linear array and the number of second LEDs, N₂, per unit length, L₂, of the second linear array fulfil N₁/L₁ < 0.5·N₂/L₂.

10. The LED filament according to any one of the preceding claims, wherein the second centroid wavelength, λ_{C2}, is in a wavelength range of 400-410 nm, and/or the first N1centroid wavelength, λ_{C1}, is in a wavelength range of 100-280 nm.

11. A LED filament arrangement, comprising,
at least one LED filament according to any one of the preceding claims,
a controller (210) coupled to the set of linear arrays, wherein the controller is configured to individually control the operation of the first linear array and the second linear array, and at least one of
a user interface (220) coupled to the controller, wherein the controller is configured to be controlled by an operator via the user interface, and
a sensor (230) coupled to the controller, wherein the sensor is configured to register sensor data and wherein the controller is configured to individually control the operation of the respective linear array of the set of linear arrays based on the sensor data.

12. The LED filament arrangement according to claim 11, wherein the sensor is configured to detect at least one of the presence of at least one person and the distance of a person to the sensor.

13. A tubular lighting device (400), comprising at least one of
the LED filament according to any one of claims 1-10, and
the LED filament arrangement according to claim 11 or 12,
the tubular lighting device (400) further comprising a tubular housing (405) comprising a first end cap (410) at a first end of the tubular housing, and a second end cap (415) at a second end of the tubular housing, opposite the first end of the tubular housing,
wherein the first and second end caps comprise a first pair and second pair of pins (420a, 420b), respectively.

14. The tubular lighting device (400) according to claim 13, further comprising a fixture (440) having first and second connectors (445a, 445b), wherein the first pair and second pair of pins of the tubular housing are configured for mating connection to the first and second connectors, respectively, for mechanical and electrical connection between the tubular housing and the fixture.

## Patentansprüche

1. Leuchtdiodenfilament, LED-Filament, (100), das konfiguriert ist, um LED-Filamentlicht (105) zu emittieren, umfassend:
einen länglichen Träger (110), der sich in einer ersten Richtung A erstreckt, umfassend eine erste Oberfläche (112) und eine zweite Oberfläche (114), die der ersten Oberfläche gegenüberliegend angeordnet ist,
eine erste lineare Anordnung (120) einer Vielzahl von ersten lichtemittierenden Dioden, LEDs, die auf der ersten Oberfläche angeordnet ist, wobei die Vielzahl der ersten LEDs so angeordnet ist, dass sie ultraviolettes Licht, UV-Licht (121) mit einer ersten Schwerpunktwellenlänge, λ_{C1}, in einem Wellenlängenbereich von 100 bis 380 nm emittiert,
eine zweite lineare Anordnung (130) einer Vielzahl von zweiten LEDs, die auf der zweiten Oberfläche angeordnet ist, **dadurch gekennzeichnet, dass**
die Vielzahl von zweiten LEDs so angeordnet ist, dass sie violettes Licht (131) mit einer zweiten Schwerpunktwellenlänge, λ_{C2}, in einem Wellenlängenbereich von 380 bis 420 nm emittiert, und
**dass**
die erste lineare Anordnung eine Anzahl, N₁, von ersten LEDs umfasst und die zweite lineare Anordnung eine zweite Anzahl, N₂, von zweiten LEDs umfasst, wobei N₁ <= 0,5 N₂.

2. LED-Filament nach Anspruch 1, wobei sich die ersten und zweiten linearen Anordnungen in einer Richtung parallel zu der ersten Richtung, A, erstrecken.

3. LED-Filament nach Anspruch 1 oder 2, ferner umfassend ein erstes Verkapselungsmittel (140), das die zweite lineare Anordnung der Vielzahl von zweiten LEDs mindestens teilweise umschließt, wobei das erste Verkapselungsmittel ein lichtstreuendes Material umfasst, das konfiguriert ist, um mindestens einen Teil des von der Vielzahl der zweiten LEDs emittierten violetten Lichts zu streuen.

4. LED-Filament nach Anspruch 3, wobei der längliche Träger lichtdurchlässig ist und wobei das lichtstreuende Material so angeordnet ist, dass es mindestens einen Teil des violetten Lichts durch den länglichen Träger streut.

5. LED-Filament nach einem der vorstehenden Ansprüche, ferner umfassend ein zweites Verkapselungsmittel (145), das die zweite lineare Anordnung der Vielzahl von zweiten LEDs mindestens teilweise umschließt, wobei das zweite Verkapselungsmittel eine reflektierende Schicht umfasst, die auf dem zweiten Verkapselungsmittel angeordnet ist, um mindestens einen Teil des von der Vielzahl der zweiten LEDs emittierten violetten Lichts zu reflektieren.

6. LED-Filament nach Anspruch 5, wobei der längliche Träger lichtdurchlässig ist und wobei die reflektierende Schicht so angeordnet ist, dass sie mindestens einen Teil des violetten Lichts durch den länglichen Träger reflektiert.

7. LED-Filament nach einem der Ansprüche 4 oder 6, wobei eines von
dem ersten Verkapselungsmittel konfiguriert ist, um mindestens 55 % des violetten Lichts durch den länglichen Träger zu streuen, und
dem zweiten Verkapselungsmittel konfiguriert ist, um mindestens 55 % des violetten Lichts durch den länglichen Träger zu reflektieren,
erfüllt wird.

8. LED-Filament nach einem der vorstehenden Ansprüche, ferner umfassend ein drittes Verkapselungsmittel (150), das die erste lineare Anordnung der Vielzahl erster LEDs mindestens teilweise umschließt, wobei das dritte Verkapselungsmittel lichtdurchlässig ist.

9. LED-Filament nach einem der vorstehenden Ansprüche, wobei die Anzahl der ersten LEDs, N₁, pro Einheitslänge, L₁, der ersten linearen Anordnung und die Anzahl der zweiten LEDs, N₂, pro Einheitslänge, L₂, der zweiten linearen Anordnung N₁/L₁ < 0,5 N₂/L₂ erfüllen.

10. LED-Filament nach einem der vorstehenden Ansprüche, wobei die zweite Schwerpunktwellenlänge, λ_{C2}, in einem Wellenlängenbereich von 400 bis 410 nm liegt und/oder die erste N1-Schwerpunktwellenlänge, λ_{C1}, in einem Wellenlängenbereich von 100 bis 280 nm liegt.

11. LED-Filamentaufbau, umfassend
mindestens ein LED-Filament nach einem der vorstehenden Ansprüche,
eine Steuerung (210), die mit dem Satz von linearen Anordnungen gekoppelt ist, wobei die Steuerung konfiguriert ist, um den Betrieb der ersten linearen Anordnung und der zweiten linearen Anordnung individuell zu steuern, und mindestens eines von
einer Benutzerschnittstelle (220), die mit der Steuerung gekoppelt ist, wobei die Steuerung konfiguriert ist, um von einem Bediener über die Benutzerschnittstelle gesteuert zu werden, und
einem Sensor (230), der mit der Steuerung gekoppelt ist, wobei der Sensor konfiguriert ist, um Sensordaten zu registrieren und wobei die Steuerung konfiguriert ist, um den Betrieb der jeweiligen linearen Anordnung des Satzes von linearen Anordnungen basierend auf den Sensordaten individuell zu steuern.

12. LED-Filamentaufbau nach Anspruch 11, wobei der Sensor konfiguriert ist, um mindestens eine der Anwesenheit mindestens einer Person und der Entfernung einer Person zum Sensor zu erkennen.

13. Röhrenförmige Beleuchtungsvorrichtung (400), umfassend mindestens eines von
dem LED-Filament nach einem der Ansprüche 1 bis 10, und
dem LED-Filamentaufbau nach Anspruch 11 oder 12,
der röhrenförmigen Beleuchtungsvorrichtung (400,) ferner umfassend ein röhrenförmiges Gehäuse (405), umfassend eine erste Endkappe (410) an einem ersten Ende des röhrenförmigen Gehäuses, und eine zweite Endkappe (415) an einem zweiten Ende des röhrenförmigen Gehäuses, gegenüber dem ersten Ende des röhrenförmigen Gehäuses,
wobei die erste und zweite Endkappe jeweils ein erstes Paar und ein zweites Paar Stifte (420a, 420b) umfassen.

14. Röhrenförmige Beleuchtungsvorrichtung (400) nach Anspruch 13, ferner umfassend eine Halterung (440) mit einem ersten und einem zweiten Verbinder (445a, 445b), wobei das erste Paar und das zweite Paar von Stiften des röhrenförmigen Gehäuses für jeweils eine passende Verbindung mit dem ersten und dem zweiten Verbinder konfiguriert sind, zur mechanischen und elektrischen Verbindung zwischen dem röhrenförmigen Gehäuse und der Halterung.

## Revendications

1. Filament à diodes électroluminescentes, DEL (100), configuré pour émettre de la lumière de filament à DEL (105), comprenant :
un support allongé (110) s'étendant dans une première direction, A, comprenant une première surface (112) et une seconde surface (114) agencée de façon opposée à la première surface,
un premier réseau linéaire (120) d'une pluralité de premières diodes électroluminescentes, DEL, agencé sur la première surface, dans lequel la pluralité de premières DEL est agencée pour émettre de la lumière ultraviolette, UV (121) avec une première longueur d'onde centroïde, λ_{C1}, dans une plage de longueur d'onde de 100 à 380 nm,
un second réseau linéaire (130) d'une pluralité de secondes DEL, agencé sur la seconde surface, **caractérisé en ce que**
la pluralité de secondes DEL est agencée pour émettre de la lumière violette (131) avec une seconde longueur d'onde centroïde, λ_{C2}, dans une plage de longueur d'onde de 380 à 420 nm, et
**en ce que**
le premier réseau linéaire comprend un nombre, N₁, de premières DEL, et le second réseau linéaire comprend un second nombre, N₂, de secondes DEL, dans lequel N₁ <= 0,5·N₂.

2. Filament à DEL selon la revendication 1, dans lequel les premier et second réseaux linéaires s'étendent dans une direction parallèle à la première direction, A.

3. Filament à DEL selon la revendication 1 ou 2, comprenant en outre un premier agent d'encapsulation (140) enfermant au moins partiellement le second réseau linéaire de la pluralité de secondes DEL, dans lequel le premier agent d'encapsulation comprend un matériau de diffusion de lumière configuré pour diffuser au moins une partie de la lumière violette émise par la pluralité de secondes DEL.

4. Filament à DEL selon la revendication 3, dans lequel le support allongé transmet la lumière, et dans lequel le matériau de diffusion de lumière est agencé pour diffuser au moins une partie de la lumière violette à travers le support allongé.

5. Filament à DEL selon l'une quelconque des revendications précédentes, comprenant en outre un deuxième agent d'encapsulation (145) enfermant au moins partiellement le second réseau linéaire de la pluralité de secondes DEL, dans lequel le deuxième agent d'encapsulation comprend une couche réfléchissante agencée sur le deuxième agent d'encapsulation pour réfléchir au moins une partie de la lumière violette émise par la pluralité de secondes DEL.

6. Filament à DEL selon la revendication 5, dans lequel le support allongé transmet la lumière, et dans lequel la couche réfléchissante est agencée pour réfléchir au moins une partie de la lumière violette à travers le support allongé.

7. Filament à DEL selon les revendications 4 ou 6, dans lequel l'un parmi
le premier agent d'encapsulation est configuré pour diffuser au moins 55 % de la lumière violette à travers le support allongé, et
le deuxième agent d'encapsulation est configuré pour réfléchir au moins 55 % de la lumière violette à travers le support allongé, est respecté.

8. Filament à DEL selon l'une quelconque des revendications précédentes, comprenant en outre un troisième agent d'encapsulation (150) enfermant au moins partiellement le premier réseau linéaire de la pluralité de premières DEL, dans lequel le troisième agent d'encapsulation transmet la lumière.

9. Filament à DEL selon l'une quelconque des revendications précédentes, dans lequel le nombre de premières DEL, N₁, par longueur unitaire, L₁, du premier réseau linéaire et le nombre de secondes DEL, N₂, par longueur unitaire, L₂, du second réseau linéaire respectent N₁/L₁ < 0,5·N₂/L₂.

10. Filament à DEL selon l'une quelconque des revendications précédentes, dans lequel la seconde longueur d'onde centroïde, λ_{C2}, est dans une plage de longueur d'onde de 400 à 410 nm, et/ou la première longueur d'onde centroïde, λ_{C1}, est dans une plage de longueur d'onde de 100 à 280 nm.

11. Agencement de filament à DEL, comprenant,
au moins un filament à DEL selon l'une quelconque des revendications précédentes,
un organe de commande (210) couplé à l'ensemble de réseaux linéaires, dans lequel l'organe de commande est configuré pour commander individuellement le fonctionnement du premier réseau linéaire et du second réseau linéaire, et au moins l'un parmi
une interface utilisateur (220) couplée à l'organe de commande, dans lequel l'organe de commande est configuré pour être commandé par un opérateur par l'intermédiaire de l'interface utilisateur, et
un capteur (230) couplé à l'organe de commande, dans lequel le capteur est configuré pour enregistrer des données de capteur et dans lequel l'organe de commande est configuré pour commander individuellement le fonctionnement du réseau linéaire respectif de l'ensemble de réseaux linéaires en fonction des données de capteur.

12. Agencement de filament à DEL selon la revendication 11, dans lequel le capteur est configuré pour détecter au moins l'une parmi la présence d'au moins une personne et la distance d'une personne par rapport au capteur.

13. Dispositif d'éclairage tubulaire (400), comprenant au moins l'un parmi
le filament à DEL selon l'une quelconque des revendications 1 à 10, et
l'agencement de filament à DEL selon la revendication 11 ou 12,
le dispositif d'éclairage tubulaire (400) comprenant en outre un logement tubulaire (405) comprenant un premier capuchon d'extrémité (410) au niveau d'une première extrémité du logement tubulaire, et un second capuchon d'extrémité (415) au niveau d'une seconde extrémité du logement tubulaire, opposée à la première extrémité du logement tubulaire,
dans lequel les premier et second capuchons d'extrémité comprennent une première paire et une seconde paire de broches (420a, 420b), respectivement.

14. Dispositif d'éclairage tubulaire (400) selon la revendication 13, comprenant en outre un bâti (440) ayant des premier et second connecteurs (445a, 445b), dans lequel la première paire et la seconde paire de broches du logement tubulaire sont conçues pour un accouplement avec les premier et second connecteurs, respectivement, pour une liaison mécanique et une connexion électrique entre le logement tubulaire et le bâti.
